# FASCICULE DE BREVET EUROPEEN

(11) **EP 2 513 291 B1**
(45) Date de publication et mention de la délivrance du brevet: **12.07.2017**
(21) Numéro de dépôt: 10808994.7
(22) Date de dépôt: 14.12.2010
(51) Int. Cl.: C12N 1/18, C12P 7/06, C12G 1/00, C12R 1/865

(54) **NOUVELLES SOUCHES DE LEVURE POUR LA PRODUCTION D'ALCOOL**
NEUE HEFESTÄMME ZUR HERSTELLUNG VON ALKOHOL
NOVEL YEAST STRAINS FOR THE PRODUCTION OF ALCOHOL

(30) Priorité: 15.12.2009 FR 0906049
(43) Date de publication de la demande: 24.10.2012
(73) Titulaire: Lesaffre et Compagnie, 75001 Paris (FR)
(72) Inventeur: BAVOUZET, Jean-Michel, F-59170 Croix (FR); LADRIERE, Jean-Marc, F-59269 Querenaing (FR); TBAIKHI, Annie, F-59118 Wambrechies (FR); WYME, William, F-59520 Marquette (FR)
(74) Mandataire: Cabinet Plasseraud
(86) Numéro de dépôt international: PCT/FR2010/000834
(87) Numéro de publication internationale: WO 2011/080411

(56) Documents cités:
- WO-A1-2009/137804
- ALMEIDA JODO R M ET AL: "Increased tolerance and conversion of inhibitors in lignocellulosic hydrolysates by Saccharomyces cerevisiae", JOURNAL OF CHEMICAL TECHNOLOGY AND BIOTECHNOLOGY, BLACKWELL SCIENTIFIC PUBLICATIONS. OXFORD, GB LNKD- DOI:10.1002/JCTB.1676, vol. 82, no. 4, 1 avril 2007 (2007-04-01), pages 340-349, XP002542370, ISSN: 0268-2575
- KADAR ZSOFIA ET AL: "Ethanol fermentation of various pretreated and hydrolyzed substrates at low initial pH", APPLIED BIOCHEMISTRY AND BIOTECHNOLOGY, vol. 137, avril 2007 (2007-04), pages 847-858, XP002590794, ISSN: 0273-2289
- NESS FREDERIQUE ET AL: "RTM1: A member of a new family of telomeric repeated genes in yeast", GENETICS, vol. 140, no. 3, 1995, pages 945-956, XP002590795, ISSN: 0016-6731

## Description

### DOMAINE TECHNIQUE

La présente invention concerne de nouvelles souches de levure et des levures issues de ces nouvelles souches destinées à la production d'alcool dans des milieux toxiques, un procédé de sélection de telles souches, ainsi qu'un procédé de production d'alcool, notamment dans des milieux toxiques.

### ARRIERE-PLAN TECHNOLOGIQUE

Il est avantageux pour un industriel de recycler les eaux issues de fermentations en vue de les utiliser dans un nouveau milieu de fermentation. Ceci permet, entre autres, de réduire les apports et les sorties en eaux des fermenteurs.

Le recyclage des eaux est effectué de façon traditionnelle après fermentation sur des milieux contenant des sources de sucres issus de betterave ou de canne, telles que les milieux EP2 (*Egout Pauvre issu de la 2^{ème} cristallisation du sucre*), mais également après fermentation sur des milieux contenant des sucres issus de blé ou de maïs.

Les eaux de recyclage permettent d'apporter des éléments nutritionnels pour la levure, mais elles présentent surtout une toxicité pour la levure, toxicité qui va augmenter au fur et à mesure que le taux de recyclage augmente.

Par ailleurs, certaines matières premières utilisées dans les milieux de fermentation sont en elles-mêmes toxiques pour la levure. C'est par exemple le cas des mélasses (appelées également EP3, ou *Egout Pauvre issu de la 3^{ème} cristallisation du sucre*).

La toxicité des eaux de recyclage et de ces matières premières toxiques résulte notamment de la présence d'acides organiques et de sels.

Dans un milieu toxique contenant partiellement ou en totalité des eaux de recyclage et/ou des matières premières toxiques, les levures connues utilisées en fermentation alcoolique ont une cinétique de production d'alcool moins rapide que dans un milieu non toxique. De plus, la quantité maximale d'alcool produit par les levures connues en fermentation dans un milieu toxique est plus faible que dans un milieu non toxique.

Lorsque les matières premières utilisées sont toxiques en elles-mêmes, leur dilution dans le milieu de fermentation permet de limiter leur effet toxique. Toutefois, cette dilution engendre des coûts supplémentaires très importants, liés notamment à une productivité volumique plus faible et des coûts de refroidissement et de distillation plus élevés.

Les tentatives de résolution du problème des milieux toxiques se sont jusqu'à présent focalisées à rendre le milieu de fermentation moins toxique. Toutefois, aucune solution réellement satisfaisante n'a été apportée à ce problème. Une autre approche a été de sélectionner des souches de levure résistantes à un milieu toxique par un procédé d'acclimatation de la levure (WO 2009/137804).

Il y a donc un réel besoin de fournir une solution alternative pour améliorer la production d'alcool par les levures dans des milieux de fermentation toxiques.

Par « amélioration de la production d'alcool », on entend une amélioration de la cinétique de production d'alcool dans un milieu de fermentation toxique, tout en maintenant une quantité acceptable d'alcool produit.

### RESUME DE L'INVENTION

Un premier objet de l'invention est de fournir une souche de *Saccharomyces cerevisiae* choisie parmi la souche déposée auprès de la CNCM sous le numéro 1-4264, la souche déposée auprès de la CNCM sous le numéro 1-4265, la souche déposée auprès de la CNCM sous le numéro 1-4411 et la souche déposée auprès de la CNCM sous le numéro 1-4412.

Un autre objet de l'invention concerne un procédé de sélection d'une souche de levure résistante à un milieu toxique comprenant :
- une étape de pré-sélection d'au moins une souche de levure dont la quantité maximale d'alcool produit, dans un milieu de sélection non toxique en condition de sucre non limitant, est supérieure ou égale à 92% de la quantité maximale d'alcool produit, dans le milieu de sélection non-toxique, par la souche de référence déposée le 4 septembre 2008 à la CNCM sous le numéro 1-4071,
- une étape de fermentation dans un milieu de sélection toxique et dans un milieu de sélection non toxique, de l'au moins une souche de levure pré-sélectionnée,
   la composition initiale du milieu de sélection toxique étant la composition initiale du milieu de sélection non toxique additionnée d'au moins un acide organique non ionisé choisi parmi l'acide acétique, acide lactique, acide formique, acide lévulinique ou un mélange de ceux-ci, et
   le sucre étant le facteur limitant de la fermentation dans le milieu de sélection toxique et dans le milieu de sélection non toxique, où la concentration de l'acide organique non ionisé dans le milieu de sélection toxique est une concentration qui entraîne une diminution de la quantité d'alcool produit à l'instant t_{max ref} par la souche de référence numéro 1-4071 de 25% à 75%, de préférence une diminution de 40% à 75%, par rapport à la quantité maximale d'alcool produite par ladite souche de référence numéro 1-4071 à l'instant t_{max ref} dans le milieu de sélection non toxique, où l'instant t_{max ref} est l'instant où la quantité maximale d'alcool produit par ladite souche de référence numéro 1-4071 est atteinte dans le milieu de sélection non toxique,
- une étape de sélection d'au moins une souche de levure dont la quantité d'alcool produit à l'instant tₘₐₓ dans le milieu de sélection toxique est supérieure ou égale à 65% de la quantité d'alcool produit à l'instant tₘₐₓ par ladite souche dans le milieu de sélection non toxique, où l'instant tₘₐₓ est l'instant où la quantité maximale d'alcool produit par ladite souche de levure est atteinte dans le milieu de sélection non-toxique, et
- une étape supplémentaire de sélection d'au moins une souche de levure dont la quantité d'alcool produit à l'instant t_{max ref} dans ledit milieu de sélection toxique est supérieure à la quantité d'alcool produit à l'instant t_{max réf} par la souche de levure de référence numéro 1-4071 dans ledit même milieu de sélection toxique.

Un autre objet de l'invention est une souche obtenue par le procédé tel que défini ci-dessus. Sont décrites également ici des souches de *Saccharomyces cerevisiae* dérivées d'une souche telle que définie ci-dessus.

Un autre objet de l'invention est une levure obtenue par culture d'une souche de levure telle que définie ci-dessus.

Un autre objet de l'invention concerne l'utilisation d'une levure telle que définie ci-dessus pour la production d'alcool.

Un autre objet de l'invention est un procédé de production d'alcool comprenant une étape de fermentation d'au moins une levure telle que définie ci-dessus, dans un milieu de fermentation.

### BREVE DESCRIPTION DES FIGURES

Figure 1 : Quantité maximale d'alcool produit à l'issue d'une fermentation dans un milieu de sélection non toxique (fermentation en sucres non limitant).
   La quantité maximale d'alcool produit (en g/kg de milieu initial) est indiquée en ordonnées pour chacun des hybrides 1 à 13 testés (axe des abscisses). « T » correspond à la souche de référence 1-4071.
Figure 2 : Impact d'un milieu de sélection toxique sur la quantité d'alcool produit à l'instant tₘₐₓ (fermentation en sucres limitant).
   L'axe des ordonnées indique la quantité d'alcool produit à l'instant tₘₐₓ dans un milieu de sélection toxique en pourcentage de la quantité d'alcool produit à l'instant tₘₐₓ dans un milieu de sélection non toxique.
   L'axe des abscisses indique les hybrides testés (1, 2, 8 et 11).
Figure 3 : Cinétique de production d'alcool en fermentation dans un milieu de sélection synthétique toxique (3,6 g/kg d'acétate à pH 4,5, fermentation en sucres limitant).
   L'axe des ordonnées indique la perte de masse (en g/kg de milieu initial) et l'axe des abscisses indique le temps en heures.
   La souche de référence 1-4071 est représentée par la courbe en pointillés avec les losanges noirs, la souche de levure 1-4264 (hybride 1) par la courbe avec les triangles, la souche de levure 1-4265 (hybride 2) par la courbe avec les carrés, l'hybride 8 par la courbe avec les cercles et l'hybride 11 par la courbe avec les losanges gris.

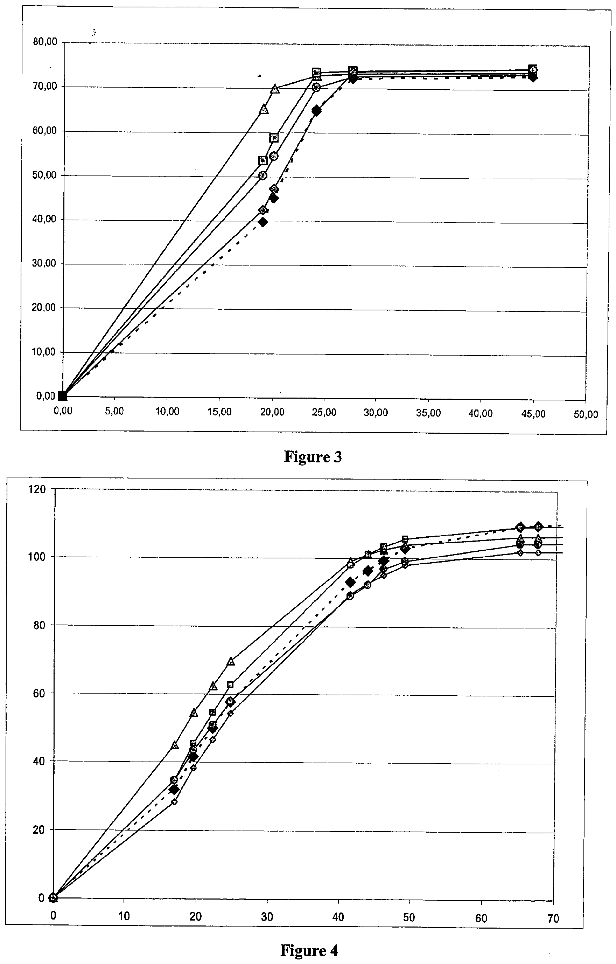

Figure 4 : Cinétique de production d'alcool en fermentation dans un milieu d'application synthétique toxique (5 g/kg d'acétate à pH 4,5, fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 (hybride 1) par la courbe avec les triangles, la souche de levure 1-4265 (hybride 2) par la courbe avec les carrés, l'hybride 8 par la courbe avec les cercles et l'hybride 11 par la courbe avec les losanges gris.

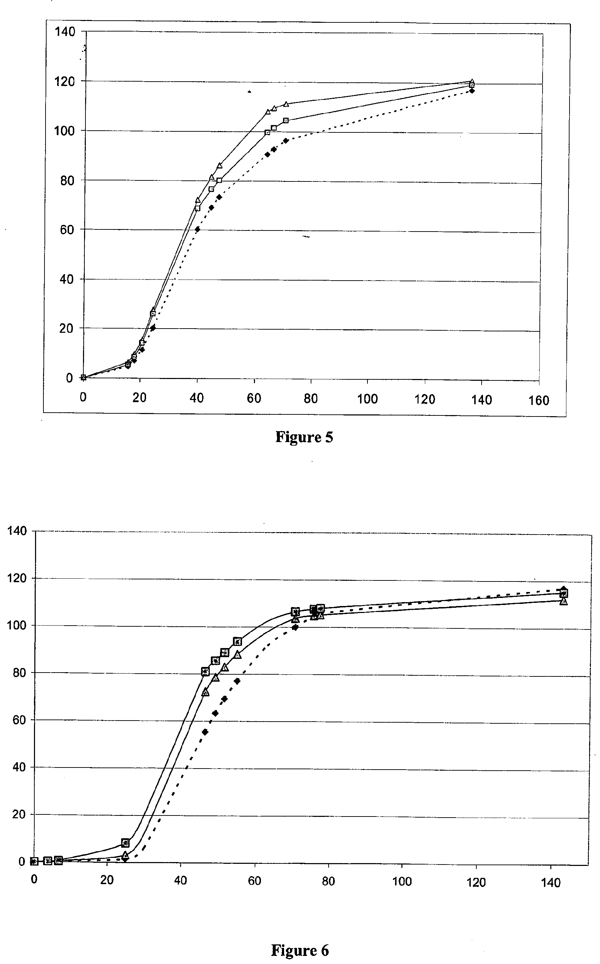

Figure 5 : Cinétique de production d'alcool en fermentation dans un milieu d'application toxique A contenant de la vinasse (fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 par la courbe avec les triangles et la souche de levure I-4265 par la courbe avec les carrés.
Figure 6 : Cinétique de production d'alcool en fermentation dans un milieu d'application toxique B contenant de la vinasse (fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 par la courbe avec les triangles et la souche de levure I-4265 par la courbe avec les, carrés.

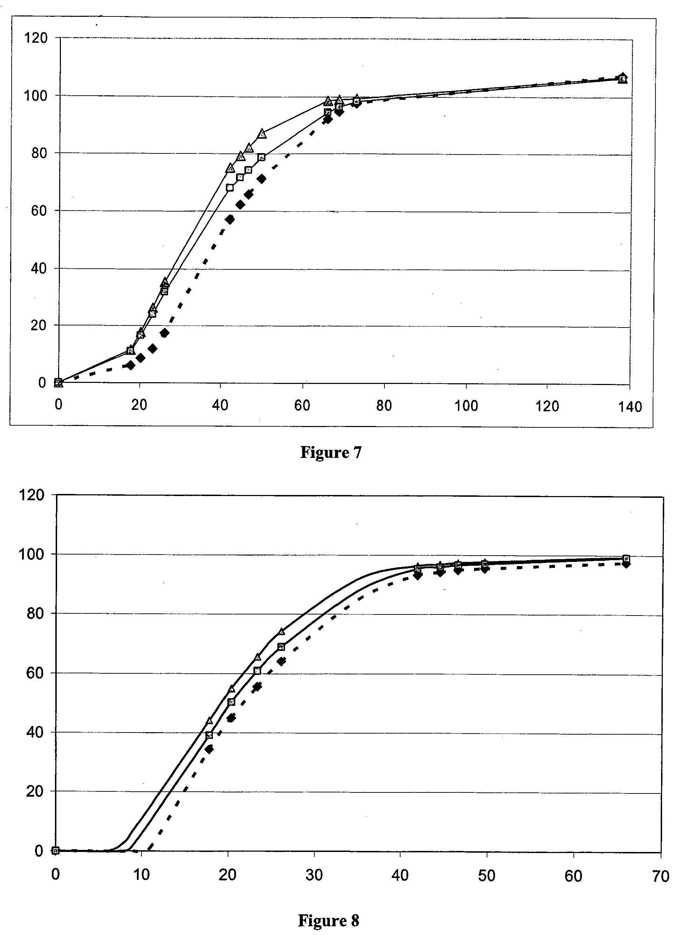

Figure 7 : Cinétique de production d'alcool en fermentation dans un milieu d'application toxique C contenant de la mélasse de betterave faiblement diluée (fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 par la courbe avec les triangles et la souche de levure I-4265 par la courbe avec les carrés.
Figure 8 : Cinétique de production d'alcool en fermentation dans un milieu d'application toxique D contenant de la mélasse de canne faiblement diluée (fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 par la courbe avec les triangles et la souche de levure I-4265 par la courbe avec les carrés.

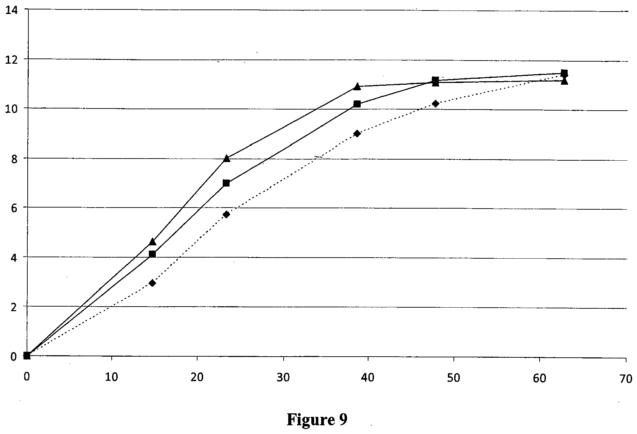

Figure 9 : Cinétique de production d'alcool en fermentation dans un milieu d'application toxique E contenant de la mélasse de la vinasse (fermentation en sucres non limitant).
   L'axe des ordonnées indique la perte de masse (en g/kg de milieu initial) et l'axe des abscisses le temps en heures.
   La souche de référence 1-4071 est représentée par la courbe en pointillés avec les losanges noirs, la souche de levure 1-4264 par la courbe avec les triangles et la souche de levure I-4265 30 par la courbe avec les carrés.

### DESCRIPTION DETAILLEE DE L'INVENTION

Les Inventeurs ont mis au point une solution alternative originale pour améliorer la production d'alcool par les levures dans des milieux de fermentation toxiques. Cette solution alternative consiste à sélectionner de nouvelles souches de levure résistantes à un milieu de fermentation toxique au moyen d'un procédé original de sélection de souches. Ainsi, en disposant d'une souche de levure résistante à un milieu de fermentation toxique, on obtient une levure résistante à un milieu de fermentation toxique.
Un milieu de fermentation toxique est un milieu de fermentation qui comprend au moins un facteur de stress.
Un facteur de stress est par exemple un stress lié à la présence d'acides organiques, un stress lié à la présence de sels, un stress lié à la présence d'autres molécules organiques inhibitrices, ou un stress lié à la pression osmotique.
Par « autres molécules organiques inhibitrices », on entend par exemple les composés aromatiques benzéniques et furaniques.
Un facteur de stress particulier est la présence d'au moins un acide organique non ionisé à une concentration totale toxique.
L'expression « concentration totale » désigne la concentration correspondant à tous les acides organiques non ionisés présents dans le milieu de fermentation.
Un « acide organique non ionisé » ou « acide organique sous forme non ionisée » est un acide carboxylique sous sa forme protonée.

Une concentration toxique en acide(s) organique(s) non ionisé(s) peut être définie par rapport à son effet toxique sur la production d'alcool d'une souche de levure de référence. Une souche de levure de référence du Demandeur pour la production d'alcool est la souche de levure déposée le 4 septembre 2008 à la CNCM sous le numéro 1-4071.
Afin d'évaluer la toxicité d'une concentration donnée en acide(s) organique(s) non ionisé(s), on compare :
- la quantité d'alcool produit par la souche de référence 1-4071, mesurée à l'instant tₘₐₓ, dans un milieu de fermentation en sucres limitant et contenant le ou les acides organiques non ionisés, et
- la quantité d'alcool produit par ladite souche de référence, mesurée à l'instant tₘₐₓ, dans un milieu de fermentation de même composition, excepté qu'il ne comprend essentiellement pas d'acide organique non ionisé.
Une concentration toxique en acide(s) organique(s) non ionisé(s) est alors par exemple une concentration entraînant une diminution de la quantité d'alcool produit à l'instant tₘₐₓ par la souche de levure numéro 1-4071 de 25% à 75% par rapport à la quantité maximale d'alcool produit par ladite souche à l'instant tₘₐₓ, dans un milieu de même composition mais ne comprenant essentiellement pas d'acide organique non ionisé, de préférence de 40% à 75%.
Ledit « milieu de fermentation contenant le ou les acides organiques non ionisés » est appelé milieu de sélection toxique par la suite.
Ledit «milieu de fermentation de même composition excepté qu'il ne comprend essentiellement pas d'acide organique non ionisé » est appelée milieu de sélection non toxique par la suite.
Le milieu de sélection non toxique comprend l'ensemble des nutriments nécessaires pour permettre une croissance cellulaire d'au moins 5 g d'équivalent levure sèche par kg de milieu.
Par «l'instant tₘₐₓ », on désigne l'instant où la quantité maximale d'alcool produit par une souche de levure est atteinte dans un milieu de sélection non toxique.

La courbe de fermentation alcoolique représentant la quantité d'alcool produit en fonction du temps comporte généralement trois phases :
- une phase de latence, au cours de laquelle il n'y a pas de production d'éthanol,
- une phase de production d'alcool, et
- une phase de plateau, qui correspond à la fin de la fermentation.
La quantité maximale d'alcool produit correspond à la quantité d'alcool produit lorsque la phase de plateau d'une fermentation alcoolique est atteinte.
Par la suite, on utilise indifféremment les termes « alcool » et « éthanol ».
Les expressions « en sucres limitant » ou « le sucre étant le facteur limitant de la fermentation » signifient que la fermentation s'arrête lorsque que les sucres fermentescibles sont totalement consommés dans le milieu de fermentation.
Les expressions « en sucres non limitant » ou « le sucre n'étant pas le facteur limitant de la fermentation » signifient que la fermentation s'arrête alors que les sucres fermentescibles ne sont pas totalement consommés dans le milieu de fermentation.
Un milieu qui ne comprend essentiellement pas d'acides organiques non ionisés est un milieu dans lequel la quantité d'alcool produit par la souche de levure numéro 1-4071 n'est pas modifiée de manière significative sur toute la durée de la fermentation par rapport à la quantité d'alcool produit par ladite souche dans un milieu de même composition, excepté qu'il ne contient aucun acide organique non ionisé.
Par exemple, un milieu comprenant une concentration inférieure ou égale à 100 mM d'acide citrique à un pH compris de 4,5 à 5,5 est un milieu ne comprenant essentiellement pas d'acide organique sous forme non ionisée.

L'expression « souche de levure » désigne une population relativement homogène de cellules de levure.
Une souche de levure est obtenue à partir de l'isolement d'un clone, un clone étant une population de cellules obtenue à partir d'une seule cellule de levure.
Une souche de levure selon l'invention est de préférence une souche de levure non génétiquement modifiée.
Par « souche de levure résistante à un milieu toxique », on désigne une souche de levure dont la cinétique de production d'alcool n'est pas trop ralentie dans un milieu de fermentation toxique.
De préférence, une souche de levure résistante à un milieu toxique a une cinétique de production d'alcool moins ralentie que celle d'une souche classique destinée à la production d'alcool, par exemple telle que la souche de levure déposée à la CNCM sous le numéro I-4071.

Afin de sélectionner de nouvelles souches de levure résistantes à un milieu toxique, les Inventeurs ont mis au point un nouveau procédé original de sélection de souches de levure. La mise en oeuvre de ce procédé a en particulier permis d'obtenir les 2 nouvelles souches de *Saccharomyces cerevisiae* déposées le 2 décembre 2009 en vertu du traité de Budapest auprès de la CNCM (Collection Nationale de Cultures de Microorganismes, Institut Pasteur, 25 rue du Docteur Roux, 75724 Paris Cedex 15, France) sous les numéros 1-4264 et 1-4265. Habituellement, lorsqu'on évalue des souches de levure destinées à la production d'alcool, on utilise comme critère de sélection la quantité maximale d'alcool produit à la fin d'une fermentation.
Le procédé de sélection de souches de levure résistantes à un milieu toxique décrit ici utilise comme critère de sélection la cinétique de production d'alcool.
Ainsi, le procédé de sélection comprend une étape de sélection des souches de levure sur la base de leur cinétique de production d'alcool dans un milieu de sélection toxique en sucres limitant par rapport à leur cinétique de production d'alcool dans un milieu de sélection non toxique en sucres limitant.
Le procédé de sélection comprend une étape supplémentaire de sélection des souches de levure sur la base de leur cinétique de production d'alcool dans un milieu de sélection toxique en sucres limitant par rapport à la cinétique de production d'alcool d'une souche de levure de référence dans ce même milieu de sélection toxique.

Le procédé de sélection comprend également une étape supplémentaire de sélection des souches de levure sur la base de leur quantité maximale d'alcool produit dans un milieu de sélection non toxique en sucres non limitant par rapport à la quantité maximale d'alcool produit par une souche de levure de référence dans le même milieu de sélection non toxique. Lorsque le procédé comprend les deux étapes supplémentaires de sélection, les différentes étapes de sélection sont effectuées dans n'importe quel ordre.
Le procédé de sélection peut comprendre en outre une étape préalable d'obtention de souches de levure par croisement et/ou mutagenèse.

Il est décrit ici un procédé de sélection d'une souche de levure résistante à un milieu toxique comprenant :
- une étape de fermentation d'au moins une souche de levure dans un milieu de sélection toxique et dans un milieu de sélection non toxique,
   la composition initiale du milieu de sélection toxique étant la composition initiale du milieu de sélection non toxique additionnée d'au moins un acide organique non ionisé dans une concentration toxique et
   le sucre étant le facteur limitant de la fermentation dans le milieu de sélection toxique et dans le milieu de sélection non toxique, et
- une étape de sélection d'au moins une souche de levure dont la quantité d'alcool produit à l'instant tₘₐₓ dans le milieu de sélection toxique est supérieur ou égal à 65% de la quantité d'alcool produit à l'instant tₘₐₓ, par ladite souche dans le milieu de sélection non toxique.

Par « composition initiale », on désigne la composition du milieu de sélection lorsque débute la fermentation.
Lorsque le milieu de sélection comprend plus d'un acide organique non ionisé, la concentration toxique est la concentration toxique totale en acides organiques non ionisés. En se plaçant « en sucres limitant », ceci permet d'être en conditions favorables pour évaluer la cinétique de production d'alcool, sans biais lié à la tolérance de la souche à l'alcool et sans biais lié à la pression osmotique initiale liée aux sucres.

La fermentation est effectuée en l'absence d'aération.
La fermentation est de préférence effectuée sous agitation.
Le pH initial des milieux de sélection toxique et non toxique sont identiques.

Le pH des milieux de sélection toxique et non toxique est tel qu'il n'inhibe pas le métabolisme de la souche de levure.
Par exemple, le pH des milieux de sélection toxique et non toxique est supérieur à 3,5 pendant la durée de la fermentation.
Dans un mode de réalisation avantageux du procédé de sélection, l'étape de fermentation est effectuée à pH constant.
L'ensemencement du milieu de sélection est identique dans le milieu de sélection toxique et dans le milieu de sélection non toxique.
De préférence, l'ensemencement est faible, afin de ne pas favoriser des souches de levure sur le milieu de sélection toxique, en raison de l'ensemencement.
L'ensemencement est par exemple compris de 0,100 g à 0,200 g de cellules de la souche de levure (en équivalent matière sèche) par kg de milieu.
La fermentation de la souche de levure est généralement effectuée à une température comprise entre 28°C et 37°C, de préférence entre 30°C à et 33°C.
De préférence, la fermentation de la souche de levure est effectuée à une température de 32°C.
Préférablement, le milieu de sélection toxique et le milieu de sélection non toxique ne contiennent pas de pentoses, de substrat lignocellulosique, d'hydrolysat ou moût de céréales, de composés aromatiques benzéniques, de composés aromatiques furaniques et/ou de mélasse.

Préférablement, la présence d'au moins un acide organique non ionisé à une concentration totale toxique est le seul facteur de stress dans le milieu de sélection toxique utilisé.
En particulier, les milieux de sélection toxique et non toxique ne présentent pas de stress lié à la présence de sels, de stress lié à la présence d'autres molécules organiques inhibitrices et/ou de stress lié à la pression osmotique.

La quantité d'alcool produit est mesurée par tout moyen approprié connu de l'homme du métier.
Il peut s'agir d'une mesure directe de l'alcool produit ou d'une mesure indirecte via un paramètre corrélé à la production d'alcool.
Par exemple, la production d'alcool peut être mesurée par chromatographie, notamment par CLHP (*Chromatographie en phase Liquide à Haute Performance*), CPG (*Chromatographie en Phase Gazeuse*), densimétrie, kit enzymatique (par exemple kit de dosage de l'éthanol de Boehringer-Mannheim R-Biopharm, Cat n° 10 176 290 035) ou un dosage par le dichromate de potassium.
La production d'alcool peut également être mesurée de manière indirecte dans un test de fermentation alcoolique par mesure de la perte de masse.
L'instant tₘₐₓ est l'instant où la quantité maximale d'alcool produit par ladite souche de levure est atteinte dans ledit milieu de sélection non toxique.

Il est décrit ici un procédé de sélection tel que défini ci-dessus, caractérisé en ce qu'il comprend :
- une étape de fermentation d'au moins une souche de levure dans un milieu de sélection toxique et dans un milieu de sélection non toxique,
   la composition initiale du milieu de sélection toxique étant la composition initiale du milieu de sélection non toxique additionnée d'au moins un acide organique non ionisé dans une concentration toxique et
   le sucre étant le facteur limitant de la fermentation dans le milieu de sélection toxique et dans le milieu de sélection non toxique, et
- une étape de sélection d'au moins une souche de levure dont la quantité d'alcool produit à l'instant tₘₐₓ dans le milieu de sélection toxique est supérieure ou égale à 70%, de préférence supérieure ou égale à 75% de la quantité d'alcool produit à l'instant tₘₐₓ par ladite souche dans le milieu de sélection non toxique.
Il est également décrit ici un procédé tel que défini ci-dessus, caractérisé en ce que le milieu de sélection toxique et le milieu de sélection non toxique sont des milieux synthétiques.
Un milieu synthétique est un milieu dont la composition chimique exacte est connue.
Un milieu synthétique comprend une source de carbone unique, une source d'azote, une source de phosphore, ainsi que les vitamines et minéraux essentiels à la croissance d'une souche de levure.
La source de carbone du milieu synthétique est un sucre fermentescible, de préférence un sucre apporté sous la forme de glucose ou saccharose.
Les sources d'azote et de phosphore sont par exemple apportées par le DAP (Di-Ammonium Phosphate ou (NH4)2 HPO4).
Le milieu synthétique peut être tamponné à un pH donné, de préférence à un pH supérieur à 3,5. Par exemple, le milieu synthétique peut comprendre de l'acide citrique comme tampon. Par exception à la définition d'un milieu synthétique, le milieu synthétique peut comprendre un extrait de levure.

Des milieux synthétiques appropriés pour la mise en oeuvre du procédé de sélection sont par exemple les milieux de sélection toxique et non toxique, en sucres limitant, décrits dans l'exemple 1.

Il est également décrit ici un procédé tel que défini ci-dessus, caractérisé en ce que l'acide organique non ionisé est choisi parmi l'acide acétique, acide lactique, acide formique, acide lévulinique ou un mélange de ceux-ci.
Préférablement, un procédé de sélection tel que défini ci-dessus, est caractérisé en ce que l'acide organique non ionisé est l'acide acétique.

Il est aussi décrit ici un procédé de sélection comprenant également une étape de sélection des souches de levure sur la base de leur cinétique de production d'alcool dans le milieu de sélection toxique par rapport à la cinétique de production d'alcool d'une souche de levure de référence dans ce même milieu de sélection toxique.
Il est également décrit ici un procédé tel que défini ci-dessus, caractérisé en ce qu'il comprend une étape supplémentaire de sélection d'au moins une souche de levure dont la quantité d'alcool produit à l'instant t_{max réf} dans ledit milieu de sélection toxique est supérieure à la quantité d'alcool produit à l'instant t_{max réf} par la souche de levure numéro 1-4071 dans ledit même milieu de sélection toxique.
L'instant t_{max réf} est l'instant tₘₐₓ de la souche de référence 1-4071, c'est-à-dire l'instant où la quantité maximale d'alcool produit par la souche de référence 1-4071 est atteinte dans le milieu de sélection non toxique.
Le milieu de sélection toxique et le milieu de sélection non toxique sont tels que définis ci-dessus, en particulier les milieux de sélection toxique et non toxique sont en condition de sucres limitant.
La quantité d'alcool produit à l'instant t_{max réf} dans ledit milieu de sélection toxique par la souche de levure sélectionnée est augmentée de préférence d'au moins 15%, de préférence d'au moins 20%, plus préférentiellement au moins 25% par rapport à la quantité d'alcool produit à l'instant t_{max réf} par la souche de levure numéro 1-4071 dans ledit même milieu de sélection toxique.

Il est également décrit ici un procédé de sélection comprenant une étape supplémentaire de sélection des souches de levure sur la base de leur quantité maximale d'alcool produit dans un milieu de sélection non toxique en sucres non limitant par rapport à la quantité maximale d'alcool produit par une souche de levure de référence dans le même milieu de sélection non toxique.
Afin de comparer les souches de levure sur la base de leur quantité maximale d'alcool produit, le sucre n'est alors pas le facteur limitant de la fermentation. Dans ce cas, c'est la concentration en alcool dans le milieu de sélection non toxique qui devient le facteur limitant de la fermentation.
Plus la quantité d'alcool produit est élevée à la fin de la fermentation, plus la souche de levure est tolérante à l'alcool.

Il est aussi décrit ici un procédé tel que défini ci-dessus, caractérisé en ce qu'il comprend une étape supplémentaire de sélection, dans un milieu de sélection non toxique dans lequel le sucre n'est pas le facteur limitant de la fermentation, d'au moins une souche de levure dont la quantité maximale d'alcool produit est supérieure ou égale à 90% de celle de la souche numéro 1-4071, de préférence supérieure ou égale à 92% de celle de la souche numéro 1-407 dans ledit même milieu de sélection non toxique. Le procédé comprend de manière avantageuse les trois étapes de sélection telles que définies ci-dessus.
Un exemple de procédé de sélection selon l'invention est détaillé dans l'exemple 1.

De manière surprenante et inattendue, le procédé de sélection de souches résistantes selon l'invention a permis d'obtenir des souches de levure résistantes à un milieu toxique qui présentent à la fois une cinétique de production d'alcool améliorée dans un milieu toxique, et une quantité maximale d'alcool produit élevée dans un milieu non toxique en sucres non limitant.
Or, les souches de levure présentent généralement soit une bonne cinétique de production d'alcool dans un milieu toxique, soit une quantité maximale d'alcool produit élevée dans un milieu non toxique en sucres non limitant, l'un de ces deux paramètres étant amélioré au détriment de l'autre.

Il est également décrit ici un procédé tel que défini ci-dessus, caractérisé en ce qu'il comprend une étape préalable d'obtention de souches de levure par croisement et/ou mutagenèse.
Le croisement de souches de levure est réalisé selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press.
Une technique classique de croisement comprend :
- une étape de sporulation de levures pour obtenir des ségrégeants,
- une éventuelle étape de sélection des ségrégeants provenant d'au moins un des deux parents sur la base de la quantité maximale d'alcool produit, et
- une étape de croisement des ségrégeants, pour obtenir des hybrides.
Les hybrides constituent de nouvelles souches de levure utilisées pour sélectionner des souches de levure résistantes à un milieu toxique.
Par le terme « mutagenèse », on désigne à la fois la mutagenèse classique obtenue par rayonnements ou par des agents chimiques mutagènes et la mutagenèse insertionnelle par transposition ou par intégration d'un fragment d'ADN exogène.
La mutagenèse par rayonnements comprend l'utilisation de rayonnements UV, X, ou gamma. Les agents chimiques mutagènes sont par exemple l'EMS (éthyl-méthyl sulfonate), l'EES (éthyl-éthyl sulfonate), la nitrosoguanidine, l'acide nitreux, l'aflatoxine B1, l'hydroxylamine, la 5-bromo uracile, la 2-amino purine, la proflavine, l'acridine orange. Dans un mode de réalisation avantageux, l'étape d'obtention de souches de levure comprend une étape de mutagenèse classique, éventuellement suivie d'un enrichissement, par exemple dans un milieu toxique.

La résistance des souches de levure 1-4264 et 1-4265 selon l'invention a été vérifiée dans un milieu synthétique toxique présentant une plus forte toxicité liée à l'acide acétique (5g/kg à pH 4,5) et présentant en outre une toxicité liée à l'alcool, du fait que le sucre n'est pas le facteur limitant de la fermentation.
Le sucre est alors apporté de préférence sous forme de dextrine pour éviter un stress osmotique lié au sucre.
Les souches de levure 1-4264 et 1-4265 ont montré un avantage cinétique par rapport à la souche de levure de référence 1-4071 dans ce milieu synthétique toxique (cf. exemple 1 et 10 figure 4).
De manière surprenante et inattendue, le procédé de sélection selon l'invention permet d'obtenir des souches de levure dont la cinétique de production d'alcool est plus rapide que celle de la souche de référence numéro 1-4071 dans un milieu d'application industrielle toxique comprenant plusieurs facteurs de stress, dont au moins un acide organique non ionisé dans une concentration toxique.

Ainsi, les souches de levure 1-4264 et 1-4265 selon l'invention ont une cinétique de production d'alcool plus rapide que celle de la souche de levure de référence 1-4071 dans des milieux de fermentation toxiques contenant de la vinasse (voir exemple 2 et figures 5 et 6) et dans des milieux de fermentation toxiques contenant des mélasses peu diluées (voir exemple 2 et figures 7 et 8).
Les souches de levure 1-4264 et 1-4265 présentent donc un réel avantage industriel dans la mise en oeuvre de fermentations alcooliques dans des milieux toxiques.
En effet, la cinétique de production d'alcool plus rapide permet d'obtenir une productivité volumique horaire plus importante des cuves de fermentation.
De plus, un autre avantage des souches de levure selon l'invention réside dans la réduction de la durée de la phase de latence lors d'une fermentation dans un milieu toxique, ce qui limite la contamination du milieu par d'autres microorganismes.
Un milieu de fermentation toxique est par exemple un milieu contenant 9% à 11% de non sucres (par rapport à la matière sèche totale du milieu), les non-sucres étant par exemple apportés par de la vinasse et/ou de la mélasse.
Par « vinasse », on désigne les eaux résiduaires de la distillation des liquides alcooliques. Les « non-sucres » comprennent les sels minéraux, les molécules organiques non consommées pendant une fermentation et les molécules organiques produites au cours d'une fermentation.

Est également décrit ici une souche de levure susceptible d'être obtenue par le procédé tel que défini ci-dessus.
La souche de levure obtenue ou susceptible d'être obtenue par le procédé tel que défini ci-dessus est de préférence une souche de levure non génétiquement modifiée.
Sont aussi décrites ici toutes les souches dérivées des souches de levure résistantes à un milieu toxique selon l'invention et qui partagent les mêmes propriétés.
Une souche de levure résistante à un milieu toxique selon l'invention est plus particulièrement une souche destinée à la production de levures utilisées en distillerie et/ou en vinification et/ou en brasserie et/ou pour la production de boissons fermentées et/ou pour la production d'alcool industriel destiné par exemple aux biocarburants ou aux industries chimiques.
Une souche de levure résistante à un milieu toxique selon l'invention est de préférence une souche de *Saccharomyces,* notamment une souche de *Saccharomyces cerevisiae*.

La présente invention a ainsi pour objet une souche de *Saccharomyces cerevisiae* choisie parmi la souche déposée auprès de la CNCM sous le numéro 1-4264 et la souche déposée auprès de la CNCM sous le numéro 1-4265.

La souche de levure 1-4264 a été obtenue par croisement de souches de levure.
La souche de levure 1-4264 produit une quantité d'alcool à l'instant tₘₐₓ dans un milieu de sélection toxique supérieure ou égale à 90% de la quantité d'alcool produit à l'instant tₘₐₓ par ladite souche dans un milieu de sélection non toxique, la composition initiale dudit milieu de sélection toxique étant la composition initiale du milieu de sélection non toxique additionnée d'au moins un acide organique non ionisé dans une concentration toxique et le sucre étant le facteur limitant de la fermentation dans le milieu de sélection toxique et dans le milieu de sélection non toxique (cf. exemple 1).
La souche de levure 1-4264 produit une quantité d'alcool à l'instant t_{max réf} dans ledit milieu de sélection toxique qui est augmentée d'au moins 50% par rapport à la quantité d'alcool produit à l'instant t_{max réf} par la souche de levure numéro 1-4071 dans ledit même milieu de sélection toxique (cf. exemple 1).
La souche de levure 1-4264 produit une quantité maximale d'alcool dans un milieu de sélection non toxique en sucres non limitant supérieure ou égale à 92% de celle de la souche numéro 1-4071 dans ledit même milieu de sélection non toxique (cf. exemple 1).

La souche de levure 1-4265 a été obtenue par croisement de souches de levure.
La souche de levure 1-4265 produit une quantité d'alcool à l'instant tₘₐₓ, dans un milieu de sélection toxique supérieure ou égale à 75% de la quantité d'alcool produit à l'instant tₘₐₓ par ladite souche dans un milieu de sélection non toxique, la composition initiale dudit milieu de sélection toxique étant la composition initiale du milieu de sélection non toxique additionnée d'au moins un acide organique non ionisé dans une concentration toxique et le sucre étant le facteur limitant de la fermentation dans le milieu de sélection toxique et le milieu de sélection non toxique (cf. exemple 1).
La souche de levure 1-4265 produit une quantité d'alcool à l'instant t_{max réf} dans ledit milieu de sélection toxique qui est augmentée d'au moins 25% par rapport à la quantité d'alcool produit à l'instant t_{max réf} par la souche de levure numéro 1-4071 dans ledit même milieu de sélection toxique (cf. exemple 1).

La souche de levure 1-4265 produit une quantité maximale d'alcool dans un milieu de sélection non toxique en sucres non limitant supérieure ou égale à 92% de celle de la souche numéro 1-4071 dans ledit même milieu de sélection non toxique (cf. exemple 1).

La présente invention a donc pour objet les deux souches décrites ci-dessus.

Est également décrite ici une souche de levure dérivée d'une souche telle que définie ci-dessus, caractérisée en ce que la quantité d'alcool produit par ladite souche dérivée à l'instant tₘₐₓ dans un milieu de sélection toxique est supérieur ou égal à 65% de la quantité d'alcool produit à l'instant tₘₐₓ par ladite souche dans un milieu de sélection non toxique, la composition initiale du milieu de sélection toxique étant la composition initiale du milieu de sélection non toxique additionnée d'au moins un acide organique non ionisé dans une concentration toxique, et le sucre étant le facteur limitant de la fermentation dans le milieu de sélection toxique et le milieu de sélection non toxique.
Une souche de *Saccharomyces cerevisiae* dérivée telle que définie ci-dessus, est caractérisée en ce que sa quantité d'alcool produit à l'instant t_{max réf} dans ledit milieu de sélection toxique est supérieure à la quantité d'alcool produit à l'instant t_{max réf} par la souche de levure numéro 1-4071 dans ledit même milieu de sélection toxique.
Une souche de *Saccharomyces cerevisiae* dérivée telle que définie ci-dessus, est caractérisée en ce que dans un milieu de sélection non toxique dans lequel le sucre n'est pas le facteur limitant de la fermentation, sa quantité maximale d'alcool produit est supérieure ou égale à 90% de celle de la souche numéro 1-4071 dans ledit même milieu de sélection non toxique. Par l'expression « souche dérivée », on désigne une souche dérivée par toute transformation quelle qu'elle soit, comme par exemple un ou plusieurs croisements et/ou une ou plusieurs mutations et/ou une ou plusieurs transformations génétiques.
Une souche dérivée par croisement peut être obtenue par croisement d'une souche selon l'invention avec la même souche, ou une autre souche selon l'invention, ou une autre souche quelconque.
Une souche dérivée par mutation peut être une souche qui a subi au moins une mutation spontanée dans son génome ou au moins une mutation induite, par exemple par mutagenèse. La ou les mutations de la souche dérivée sont silencieuses ou non.
Une souche dérivée par transformation génétique est une souche dans laquelle a été introduit un ADN exogène.
Ledit ADN exogène peut être apporté par un plasmide.

Ledit ADN exogène est de préférence intégré dans le génome de la levure.
Une souche dérivée peut également être un clone isolé à partir d'une culture d'une souche de levure selon l'invention.
La présente invention a ainsi pour objet les souches de *Saccharomyces cerevisiae* déposées le 8 décembre 2010 en vertu du traité de Budapest auprès de la CNCM (*Collection Nationale de Cultures de Microorganismes, Institut Pasteur, 25 rue du Docteur Roux, 75724 Paris Cedex 15, France*) sous les numéros 1-4411 et 1-4412.
La souche de levure 1-4411 a été obtenue par isolement d'un clone à partir d'une culture de la souche 1-4264.
La souche de levure 1-4411 produit une quantité d'alcool à l'instant tₘₐₓ dans un milieu de sélection toxique supérieure ou égale à 95% de la quantité d'alcool produit à l'instant tₘₐₓ par la souche dans un milieu de sélection non toxique, la composition initiale dudit milieu de sélection toxique étant la composition initiale du milieu de sélection non toxique additionnée d'au moins un acide organique non ionisé dans une concentration toxique et le sucre étant le facteur limitant de la fermentation dans le milieu de sélection toxique et dans le milieu de sélection non toxique (cf. exemple 1).
De manière surprenante, la cinétique de production d'alcool de la souche de levure 1-4411 n'est pas altérée dans un milieu de sélection toxique par rapport au milieu de sélection non toxique.
La souche de levure 1-4411 produit une quantité d'alcool à l'instant t_{max réf} dans ledit milieu de sélection toxique qui est augmentée d'au moins 12% par rapport à la quantité d'alcool produit à l'instant t_{max réf} par la souche de levure numéro 1-4071 dans ledit même milieu de sélection toxique (cf. exemple 1).
La souche de levure 1-4411 produit une quantité d'alcool dans un milieu de sélection non toxique en sucres non limitant supérieure ou égale à 95% de celle de la souche numéro I-4071 dans ledit même milieu de sélection non toxique (cf. exemple 1).

La souche de levure 1-4412 a été obtenue par isolement d'un clone à partir d'une culture de la souche 1-4264.
La souche de levure 1-4412 produit une quantité d'alcool à l'instant tₘₐₓ dans un milieu de sélection toxique supérieure ou égale à 72% de la quantité d'alcool produit à l'instant tₘₐₓ par ladite souche dans un milieu de sélection non toxique, la composition initiale dudit milieu de sélection toxique étant la composition initiale du milieu de sélection non toxique additionnée d'au moins un acide organique non ionisé dans une concentration toxique et le sucre étant le facteur limitant de la fermentation dans le milieu de sélection toxique et dans le milieu de sélection non toxique (cf. exemple 1).
La souche de levure 1-4412 produit une quantité d'alcool à l'instant t_{max réf} dans ledit milieu de sélection toxique supérieure ou égale à 90% de la quantité d'alcool produit à l'instant t_{max réf} par la souche de levure numéro 1-4071 dans ledit même milieu de sélection toxique (cf. exemple 1).
La souche de levure 1-4412 produit une quantité maximale d'alcool dans un milieu de sélection non toxique en sucres non limitant supérieure ou égale à 95% de celle de la souche numéro 1-4071 dans ledit même milieu de sélection non toxique (cf. exemple 1).

De plus, les souches de levure 1-4411 et 1-4412 ont une cinétique de production d'alcool plus rapide que celle de la souche de levure de référence 1-4071 dans des milieux de fermentation toxiques contenant de la vinasse (voir exemple 2 et figure 9).
Les souches de levure 1-4411 et 1-4412 présentent donc un réel avantage industriel dans la mise en oeuvre de fermentations alcooliques dans des milieux toxiques.

Est également décrit ici un procédé de transformation d'une souche de levure résistante à un milieu toxique, pour obtenir une souche dérivée telle que définie ci-dessus, ledit procédé de transformation comprenant une étape de transformation de ladite souche par au moins un croisement et/ou au moins une mutation et/ou au moins une transformation génétique.

La présente invention a particulièrement pour objet une levure obtenue par culture d'une souche de levure telle que définie ci-dessus.
Les levures selon l'invention sont particulièrement intéressantes dans les applications suivantes :
- l'alcool dit «de bouche », destiné à la fabrication de boissons alcoolisées, et/ou
- l'alcool industriel, destiné par exemple aux biocarburants ou aux industries chimiques.
Les levures sont produites à partir de souches de levure résistantes à un milieu toxique selon l'invention, notamment comme décrit dans le livre de référence « Yeast Technology », 2ème édition, 1991, G. Reed et T.W. Nagodawithana, publié par Van Nostrand Reinhold, ISBN 0-442-31892-8.
La multiplication des levures, à l'échelle industrielle, comprend en général au moins les deux premières étapes de l'ensemble des étapes suivantes :
- multiplication d'une souche de levure en plusieurs stades, d'abord en semianaérobiose, puis en aérobiose,
- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,
- filtration de la crème de levure liquide ainsi obtenue, en général sur un filtre rotatif sous vide, pour obtenir une levure fraîche déshydratée contenant de 26% à 35% de matière sèche,
- malaxage de ladite levure fraîche déshydratée, pour obtenir une masse homogène,
- extrusion de la levure ainsi obtenue, pour obtenir
   - une levure pressée sous forme de pains de levure fraîche ou de levure fraîche émiettée, contenant environ 30% de matière sèche, ou
   - une levure sous forme de particules, en général de granules, si la levure est destinée à être séchée,
- éventuellement, séchage de manière ménagée, dans un courant d'air chaud, par exemple par fluidisation, des particules de levures obtenues par extrusion pour obtenir de la levure sèche.
L'étape de séchage est de préférence un séchage rapide ménageant en présence d'un émulsifiant.
Parmi les émulsifiants pouvant être utilisés lors de l'étape de séchage, on peut choisir le monostéarate de sorbitan, utilisé par exemple à une concentration d'environ 1,0% (en poids sur le poids de levure sèche).
Les levures selon l'invention sont des levures résistantes à un milieu toxique.
Les levures selon l'invention peuvent être utilisées sous toute forme possible.
Par exemple, une levure telle que définie ci-dessus, peut être caractérisée en ce qu'elle est sous forme de crème de levure, de levure pressée, de levure sèche ou de levure surgelée.

La présente invention a également pour objet l'utilisation d'une levure telle que définie ci-dessus pour la production d'alcool.
La production d'alcool est obtenue par fermentation alcoolique.
Les conditions de fermentation alcoolique dépendent du type d'application recherchée, par exemple selon qu'il s'agit d'une fermentation de type brasserie, vinification ou distillerie.

L'homme du métier sait déterminer les conditions appropriées pour une fermentation alcoolique.
Le milieu de fermentation alcoolique comprend les éléments suivants : au moins une source de carbone fermentescible, au moins une source d'azote, au moins une source de soufre, au moins une source de phosphore, au moins une source de vitamines et/ou au moins une source de minéraux.
La source de carbone est par exemple apportée sous la forme d'un sucre immédiatement assimilable par la levure, glycérol, éthanol et/ou un mélange de ceux-ci.
La source de carbone est de préférence un sucre immédiatement assimilable par la levure, par exemple un sucre simple de type glucose ou fructose, ou un disaccharide de type saccharose et/ou un mélange de ces sucres.
Le sucre peut être apporté sous la forme de sirops de glucose et/ou de sirops de fructose et/ou d'hydrolysats d'amidon et/ou sous la forme de mélasses et/ou sous la forme d'EP2 (*Egout Pauvre issu de la 2^{ème} cristallisation du sucre*) et/ou sous la forme d'une substance capable de donner des sucres assimilables par la souche de levure et/ou sous la forme d'un mélange de ceux-ci.
La source d'azote est par exemple apportée sous la forme de sulfate d'ammonium, hydroxyde d'ammonium, di-ammonium phosphate, ammoniac, urée et/ou une combinaison de ceux-ci. La source de soufre est par exemple apportée sous la forme de sulfate d'ammonium, sulfate de magnésium, acide sulfurique et/ou une combinaison de ceux-ci.
La source de phosphore est par exemple apportée sous la forme d'acide phosphorique, phosphate de potassium, di-ammonium phosphate, mono-ammonium phosphate, et/ou une combinaison de ceux-ci.
La source de vitamines est par exemple apportée sous la forme de mélasse, hydrolysat de levure, solution de vitamine pure ou d'un mélange de vitamines pures et/ou une combinaison de ceux-ci.
La source de vitamines apporte à la levure l'ensemble des vitamines dans des quantités au moins équivalentes à celles recommandées dans les ouvrages de référence. Plusieurs sources de vitamines peuvent être associées.
La source de minéraux est par exemple apportée sous la forme de mélasse, un mélange de sels minéraux et/ou leur combinaison.
La source de minéraux apporte à la levure l'ensemble des macroéléments et oligoéléments dans des quantités au moins équivalentes à celles recommandées dans les ouvrages de référence. Plusieurs sources de minéraux peuvent être associées. Une même substance peut apporter plusieurs éléments différents.

A titre d'exemple, on peut se référer aux conditions de fermentation alcoolique décrites dans le livre de référence « Yeast Technology », 2ème édition, 1991, G. Reed et T.W. 10 Nagodawithana, publié par Van Nostrand Reinhold, ISBN 0-442-31892-8.
En particulier est décrit ici l'utilisation d'une levure telle que définie ci-dessus pour la production d'alcool dans un milieu de fermentation toxique.

Un procédé de production d'alcool tel que décrit ici comprend généralement une étape de fermentation d'au moins une levure telle que définie ci-dessus, dans un milieu de fermentation.
Le milieu de fermentation est tel que défini ci-dessus.

Le procédé de production d'alcool tel que défini ci-dessus, est caractérisé en ce que ledit milieu de fermentation est un milieu de fermentation toxique.

En particulier, le procédé de production d'alcool tel que défini ci-dessus, est caractérisé en ce que ledit milieu de fermentation toxique comprend une eau recyclée et/ou de la mélasse. Une eau recyclée est par exemple de la vinasse, une eau de trempage de grains, de la flegmasse, une eau de condensation des gaz de fermentation, ou une eau issue de la concentration de vinasse.
Le pourcentage d'eau recyclée, vinasse et/ou mélasse dans le milieu de fermentation toxique dépend des conditions de fermentation.
Le milieu de fermentation comprend par exemple de 20% à 40% de vinasse.

Le procédé de production d'alcool tel comprend généralement une étape de recyclage partiel ou total du milieu de fermentation.
Le recyclage du milieu de fermentation peut être effectué en continu ou à différents intervalles de temps au cours de la fermentation.
Lors d'un procédé de production d'alcool en continu avec cascade de fermenteurs, le recyclage du milieu de fermentation peut être effectué dans la ou les premières cuves de fermentation.

La présente invention va maintenant être illustrée à l'aide des exemples suivants qui sont donnés à titre d'illustration et ne sont nullement limitatifs. Les exemples décrivent en particulier un procédé de sélection de souches de levure selon l'invention résistantes à un milieu toxique et leurs caractéristiques.

### EXEMPLE 1: SELECTION DE SOUCHES DE LEVURE RESISTANTES A UN MILIEU TOXIQUE

### Matériel et Méthodes

### 1. Milieux

| | | | |
|---|---|---|---|
| **Milieu YEG** | | | |
| Glucose | 20 g | | |
| Extrait de levure | 5 g | | |
| Agar | 30 g | | |
| Eau | qsp 11 | | |
| | | | |
| **Milieu de sélection toxique** (sucres limitant) | | **Milieu de sélection non toxique** (sucres limitant) | |
| Glucose | 150 g | Glucose | 150 g |
| Extrait de levure | 5 g | Extrait de levure | 5 g |
| (NH4)2 HP04 | 4,70 | (NH4)2 HPO4 | 4,70 |
| Acide acétique | 2,52 g | Acide citrique | 11,40 g |
| Acétate de sodium | 2,08 g | Citrate de sodium | 13,50 g |
| Acide citrique | 11,40 g | Vitamines | |
| Citrate de sodium | 13,50 g | Sels minéraux | |
| Vitamines | | Eau | qsp 1kg |
| Sels minéraux | | pH 4,5 | |
| Eau | qsp 1 kg | | |
| pH 4,5 | | | |
| **Milieu d'application synthétique toxique** (sucres non limitant) | | **Milieu de sélection non toxique** (sucres non limitant) | |
| Dextrine | 307,3 g | Glucose | 300 g |
| Extrait de levure | 5 g | Extrait de levure | 5 g |
| KH2PO4 | 1 g | KH2PO4 | 1 g |
| Urée | 2,5 g | Urée | 2,5 g |
| Acide citrique | 0,36 g | Acide citrique | 0,36 g |
| Citrate | 0,53 g | Citrate | 0,53 g |
| Acide acétique Vitamines Sels minéraux | 5 g | Acide acétique Vitamines Sels minéraux | 2,50 g |
| Eau | qsp 1kg | Eau | qsp 1kg |
| pH 4,5 | | pH 5,5 | |
| | | | |
| + amyloglucosidase ajoutée extemporanément | | | |
| **Milieu de pré**-**culture** | | **Milieu de sporulation** | |
| Saccharose | 100 g | Acétate de sodium | 6,5 g |
| Extrait de levure | 20 g | Agar | 15 g |
| Sels minéraux | | Eau | qsp 1L |
| Eau | qsp 11 | pH 6,5 | |

Tous les milieux utilisés sont stérilisés.

### 2. Croisement pour l'obtention d'hybrides

Les souches de levure sont mises à sporuler sur le milieu de sporulation pendant 3 à 5 jours à 30°C. Les cellules de levure sont récupérées dans de l'eau stérile. Elles sont ensuite digérées par une 13-glucanase préparée extemporanément à 20 mg/ml, pendant 30min, à 30°C et sous agitation.

Les cellules de levure sont ensuite étalées sur une gélose de milieu YEG. Les spores sont alors disséquées à l'aide d'un micromanipulateur. Les spores sont cultivées à 30°C pendant 2 jours.

Les spores des souches parentes sont mélangées sur une gélose de milieu YEG. L'hybridation est effectuée pendant 3 à 5 jours à 30°C. Les mélanges sont alors étalés sur une gélose de milieu YEG. Après 48h d'incubation à 30°C, des colonies apparaissent et des PCR sont 15 effectuées sur ces colonies afin d'identifier les hybrides (Huxley et al., 1990).

### 3. Fermentation

Les cellules de levure sont pré-cultivées dans 10ml de milieu de pré-culture ajusté à pH 4,7 pendant 24h à 30°C.

2mL de cette pré-culture sont inoculés dans 200ml de milieu de pré-culture pendant 20h à 26°C sous agitation.

Les cellules de levures sont alors récupérées par centrifugation pendant 5min à 4500rpm et à 4°C. Les cellules de levure sont lavées avec de l'eau froide et sont à nouveau centrifugées dans les mêmes conditions. Les cellules de levure sont récupérées dans 40ml d'eau froide. La matière sèche obtenue est mesurée.

La fermentation est réalisée à 32°C, en l'absence d'aération, sous agitation (95rpm).

Le milieu de fermentation (milieu de sélection toxique ou milieu de sélection non toxique) est ensemencé à 0,125g de levures (en équivalent matière sèche) par kg de milieu.

### 4. Mesure de la quantité d'alcool produit - Méthode directe

La quantité d'éthanol est déterminée par chromatographie liquide haute performance (HPLC) 15 (Shimadzu®) en utilisant une colonne Aminex HPX 87H (Biorad®), résine échangeuse de protons, éluée par une solution d'acide sulfurique 5 mM.

### 5. Evaluation de la quantité d'alcool produit - Méthode indirecte

La quantité d'alcool produit étant corrélée à la perte de masse du milieu de fermentation, la quantité d'alcool produit est évaluée par perte de masse du milieu de fermentation. Après calcul, on obtient la quantité d'alcool produit (en g/kg de milieu initial).

### Résultats

### 1. Croisement

Plusieurs souches de levure de la collection de culture du Demandeur sont utilisées comme souches parentes pour les croisements.

### 2. Pré-sélection des hybrides sur la quantité maximale d'alcool produit dans un milieu de sélection non toxique

Parmi les hybrides obtenus à l'étape de croisement, 59 hybrides sont ensuite testés sur la base de la concentration maximale d'alcool produit dans un milieu de sélection non toxique, en condition de sucre non limitant.

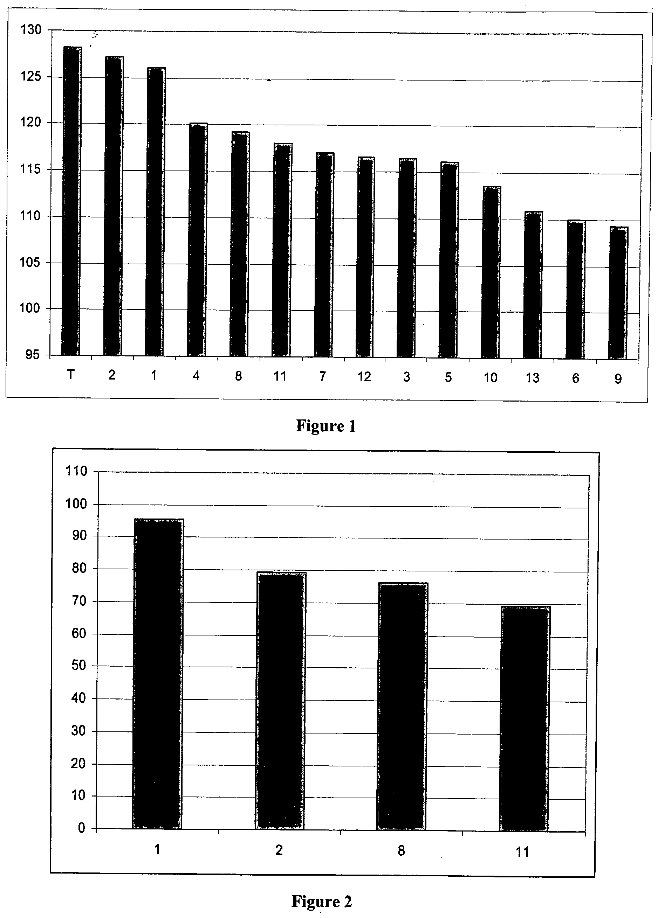

La figure 1 montre la quantité d'alcool produit dans le milieu de sélection non toxique (en g/kg) à l'issue de la fermentation pour les 13 meilleurs hybrides.

La quantité finale d'alcool produit par les hybrides est comparée à celle de la souche de référence 1-4071 (notée T dans la figure 1).

Les hybrides dont la quantité maximale d'alcool produit est supérieure ou égale à 92% de celle de la souche de référence 1-4071 sont pré-sélectionnés. Il s'agit des hybrides 1, 2, 4, 8 et 11.

### 3. Sélection des hybrides sur la base de leur cinétique de production d'alcool dans un milieu de sélection toxique par rapport à un milieu de sélection non toxique

Les hybrides 1, 2, 4, et 8 sont ensuite testés sur la base de leur cinétique de production d'alcool dans le milieu de sélection toxique par rapport au milieu de sélection non toxique. Les résultats sont présentés dans la figure 2 pour les hybrides 1, 2, 8 et 11.

L'hybride 4 présentant un problème de croissance sur le milieu de sélection toxique, il n'est pas représenté dans la figure 2.

A son instant tₘₐₓ, l'hybride 1 produit une quantité d'alcool dans le milieu de sélection toxique qui est supérieure ou égale à 90% de la quantité d'alcool produit dans le milieu de sélection non toxique.

A son instant tₘₐₓ, l'hybride 2 produit une quantité d'alcool dans le milieu de sélection toxique qui est supérieure ou égale à 75% de la quantité d'alcool produit dans le milieu de sélection non toxique.

A son instant tₘₐₓ, l'hybride 8 produit une quantité d'alcool dans le milieu de sélection toxique qui est supérieure ou égale à 70% de la quantité d'alcool produit dans le milieu de sélection non toxique.

A son instant tₘₐₓ, l'hybride 11 produit une quantité d'alcool dans le milieu de sélection toxique qui est supérieure ou égale à 65% de la quantité d'alcool produit dans le milieu de sélection non toxique.

Par comparaison, à son instant tₘₐₓ, la souche de levure de référence 1-4071 produit une quantité d'alcool dans le milieu de sélection égale à 60 % de la quantité d'alcool produit dans le milieu de sélection non toxique.

Les hybrides 1, 2, 8 et 11 sont sélectionnés au cours de cette étape.

### 4. Sélection des hybrides sur la base de leur cinétique de production d'alcool dans un milieu de sélection toxique par rapport à la souche de référence I-4071

Les hybrides 1, 2, 8 et 11 sont ensuite testés en fermentation dans le milieu de sélection toxique par rapport à la souche de levure de référence 1-4071.
La figure 3 montre que la cinétique de production d'alcool des hybrides 1, 2 et 8 est plus rapide que celle de la souche 1-4071.
La cinétique de production d'alcool de l'hybride 11 est proche de celle de la souche de référence 1-4071.
De plus, l'hybride 1 produit une quantité d'alcool à l'instant t_{max ref} dans le milieu de sélection toxique qui est augmentée d'au moins 50% par rapport à la quantité d'alcool produit à l'instant t_{max ref} par la souche de levure numéro I-4071 dans ce milieu de sélection toxique. S'agissant de l'hybride 2, il produit une quantité d'alcool à l'instant t_{max ref} dans le milieu de sélection toxique qui est augmentée d'au moins 25% par rapport à la quantité d'alcool produit à l'instant t_{max ref} par la souche de levure numéro 1-4071 dans ce milieu de sélection toxique.

S'agissant de l'hybride 8, il produit une quantité d'alcool à l'instant t_{max ref} dans le milieu de sélection toxique qui est augmentée d'au moins 20% par rapport à la quantité d'alcool produit à l'instant t_{max ref} par la souche de levure numéro 1-4071 dans ce milieu de sélection toxique.

La résistance des hybrides 1, 2, 8 et 11 est alors testée dans le milieu d'application synthétique toxique présentant une plus forte toxicité liée à l'acide acétique (5g/kg à pH 4,5) et présentant en outre une toxicité liée à l'alcool, du fait que le sucre est ajouté en quantité non limitante de la fermentation.
Le sucre est apporté sous forme de dextrines pour éviter un stress osmotique lié au sucre. Comme indiqué dans la figure 4, seules les souches de levure 1-4264 et 1-4265 ont montré un avantage cinétique par rapport à la souche de levure de référence 1-4071 dans ce milieu d'application synthétique toxique.

En conclusion, les hybrides 1 et 2 ont un avantage cinétique par rapport à la souche de référence 1-4071 lors de la production d'alcool dans le milieu de sélection synthétique toxique. Les hybrides 1 et 2 correspondent respectivement à la souche de levure 1-4264 et I-4265 selon l'invention.

### 5. Isolement de clones à partir d'une culture de la souche de levure I-4264

Une culture de la souche de levure 1-4264 est réalisée dans le milieu YEG.
Un étalement sur boîte de la culture est ensuite effectué. Plusieurs clones sont sélectionnés et leur cinétique de production d'alcool est évaluée, ainsi que la quantité maximale d'alcool produite dans le milieu de sélection non toxique en sucres non limitant.
Les résultats obtenus concernant les clones A et C sont indiqués dans le tableau 1.

**Tableau 1**

| **Clone** | **Quantité d'alcool produit à tₘₐₓ dans le milieu toxique / milieu non toxique (sucres limitant)** | **Quantité d'alcool produit par rapport à la souche I-407 à tₘₐₓ réf dans le milieu toxique (sucres limitant)** | **Quantité maximale d'alcool produit dans le milieu de sélection non toxique par rapport à la souche I-4071 (sucres non limitant** |
|---|---|---|---|
| **Clone A** | 99% | + 15% | 98.6% |
| **Clone C** | 76% | -10% | 98.7% |

Les clones A et C ont une quantité maximale d'alcool produit supérieure ou égale à 95% de celle de la souche de référence 1-4071 dans un milieu de sélection non toxique, en condition de sucres non limitant.
A son instant tₘₐₓ, le clone A produit une quantité d'alcool dans le milieu de sélection toxique qui est supérieure ou égale à 95% de la quantité d'alcool produit dans le milieu de sélection non toxiques (en sucres limitant).
A son instant tₘₐₓ, le clone C produit une quantité d'alcool dans le milieu de sélection toxique qui est supérieure ou égale à 72% de la quantité d'alcool produit dans le milieu de sélection non toxique (en sucres limitant).
Les clones sont ensuite évalués en fermentation dans le milieu de sélection toxique (en sucres limitant) par rapport à la souche de levure de référence 1-4071.
La cinétique de production d'alcool du clone A est plus rapide que celle de la souche 1-4071. Le clone A produit ainsi une quantité d'alcool à l'instant t_{max ref} dans le milieu de sélection toxique qui est augmentée d'au moins 12% par rapport à la quantité d'alcool produit à l'instant t_{max ref} par la souche de levure numéro 1-4071 dans ce milieu de sélection toxique. Le clone A présente un réel avantage cinétique par rapport à la souche de référence 1-4071 lors de la production d'alcool dans le milieu de sélection synthétique toxique.
La cinétique de production d'alcool du clone C est légèrement en retrait par rapport à celle de la souche de référence 1-4071.
Le clone C produit une quantité d'alcool à l'instant t_{max ref} dans le milieu de sélection toxique supérieure ou égale à 90% de la quantité d'alcool produite à l'instant t_{max ref} par la souche de levure numéro 1-4071 dans ce milieu de sélection toxique.

Les clones A et C correspondent respectivement aux souches de levure 1-4411 et 1-4412.

### EXEMPLE 2: UTILISATION DES SOUCHES DE LEVURE SELON L'INVENTION EN MILIEU D'APPLICATION TOXIQUE

### Matériel et Méthodes

### 1. Milieux

| | | | |
|---|---|---|---|
| **Milieu A** | | **Milieu B** | |
| - EP2 de betterave | 416,2 g | - EP2 de betterave | 373,1 g |
| - vinasse | 118,1 g | - vinasse | 572,9 g |
| - urée | 1,6 g | - urée | 1,6 g |
| - MgSO4 7H2O à 9,5% | 0,1 g | - MgSO4 7H2O à 9,5% | 0,1 g |
| - H3PO4 à 75% | 0,5 g | - H3PO4 à 75% | 0,5 g |
| - eau | qsp 1kg | - Bioflorure d'ammonium à 3,5% | 1,4 g |
| - pH initial 5 | | - eau | qsp 1kg |
| | | - pH initial 5 | |
| **Milieu C** | | **Milieu D** | |
| - Mélasse de betterave | 394,7 g | - Mélasse de canne | 315,2 g |
| - (NH4)2 HPO4 | 4,7 g | - glucose | 68,4 g |
| - vitamines | | - (NH4)2 HPO4 | 4,7 g |
| - eau | qsp 1 kg | - vitamines | |
| - pH initial 5,3 | | - eau | qsp 1 kg |
| | | - pH initial 5,3 | |
| **Milieu E** | | | |
| - EP2 de betterave | 353,1 g | | |
| - vinasse | 413,2 g | | |
| - urée | 0,39 g | | |
| - (NH4)2 HPO4 | 0,13 g | | |
| - Magnésium et zinc | | | |
| - Eau de procédé* | 233,6 g | | |
| - pH initial 5 | | | |
| * *mélange comprenant de l'eau issue des cycles de nettoyage, des condensats de fermentation et de distillation* | | | |

Le milieu A contient 9% de non sucres (par rapport à la matière sèche totale du milieu), les non-sucres étant en grande majorité apportés par la vinasse.
Le milieu B contient 9% de non sucres (par rapport à la matière sèche totale du milieu), les non-sucres étant en grande majorité apportés par la vinasse.
Le milieu C contient 10,8% de non sucres (par rapport à la matière sèche totale du milieu), les non-sucres étant apportés par la mélasse (EP3) de betterave qui est faiblement diluée.
Le milieu D contient 10,8% de non sucres (par rapport à la matière sèche totale du milieu), les non-sucres étant apportés par la mélasse (EP3) de canne qui est faiblement diluée.
Le milieu E contient 11% de non sucres (par rapport à la matière sèche totale du milieu), les non-sucres étant en grande majorité apportés par la vinasse.

### 2. Fermentation et mesure de l'alcool

Les conditions de fermentation sont les mêmes que dans l'exemple 1, excepté que le milieu de fermentation contient du sucre en quantité non limitante.

La quantité d'alcool produit est mesurée par perte de poids, comme détaillé dans l'exemple 1.

### Résultats

Les hybrides 1 et 2 sélectionnés et correspondant respectivement aux souches de levure I-4264 et 1-4265 sont ensuite testés dans des milieux d'application toxiques.

Les milieux d'application toxiques testés sont les suivants :
- les milieux A et B contenant une quantité importante de vinasse, et
- les milieux C et D contenant de la mélasse faiblement diluée, respectivement de betterave et de canne.

Les clones A et C correspondant respectivement aux souches de levure 1-4411 et 1-4412 sont testés dans le milieu d'application toxique E.

Les figures 5 à 8 montrent la cinétique de production d'alcool des souches de levure 1-4264 et 1-4265 selon l'invention et de la souche de référence 1-4071, respectivement dans les milieux A, B, C et D.

La figure 9 montre la cinétique de production d'alcool des souches de levure 1-4411 et I-4412 et de la souche de référence 1-4071 dans le milieu d'application toxique E.

Comme indiqué dans la figure 5, la cinétique de production d'alcool des souches de levure I-4264 et 1-4265 selon l'invention est plus rapide que celle de la souche de référence 1-4071 après 20h de fermentation. L'écart entre la quantité d'alcool produit par les hybrides et la quantité d'alcool produit par la souche de référence s'accentue à partir de 20h jusqu'à la fin de la fermentation.

Comme indiqué dans la figure 6, la cinétique de production d'alcool des souches de levure I-4264 et 1-4265 est plus rapide que celle de la souche de référence 1-4071, respectivement après 20h de fermentation et après 30 heures de fermentation. La phase de latence de la souche 1-4264 est très courte en comparaison des 2 autres souches. L'écart entre la quantité d'alcool produit par les hybrides et la quantité d'alcool produit par la souche de référence s'accentue à partir de 20h et 30h respectivement jusqu'à la fin de la fermentation.

Comme indiqué dans les figures 7 et 8, la cinétique de production d'alcool des souches de levure 1-4264 et 1-4265 est plus rapide que celle de la souche de référence 1-4071 sur toute la durée de la fermentation.

Comme indiqué dans la figure 9, la cinétique de production d'alcool des souches de levure I-4411 et 1-4412 est plus rapide que celle de la souche de référence 1-4071 sur toute la durée de la fermentation dans le milieu d'application E.

En conclusion, la souche de référence 1-4071 est plus affectée par la toxicité des milieux d'application testés que les souches de levure 1-4264, 1-4265, 1-4411 et 1-4412 selon l'invention.

Les souches de levure 1-4264, 1-4265, 1-4411 et 1-4412 selon l'invention procurent un avantage cinétique pour la production d'alcool en milieu toxique.

## Revendications

1. Procédé de sélection d'une souche de levure résistante à un milieu toxique comprenant:
- une étape de pré-sélection d'au moins une souche de levure dont la quantité maximale d'alcool produit, dans un milieu de sélection non toxique en condition de sucre non limitant, est supérieure ou égale à 92% de la quantité maximale d'alcool produit, dans le milieu de sélection non-toxique, par la souche de référence déposée le 4 septembre 2008 à la CNCM sous le numéro 1-4071,
- une étape de fermentation, dans un milieu de sélection toxique et dans un milieu de sélection non toxique, de l'au moins une souche de levure pré-sélectionnée,
la composition initiale du milieu de sélection toxique étant la composition initiale du milieu de sélection non toxique additionnée d'au moins un acide organique non ionisé choisi parmi l'acide acétique, acide lactique, acide formique, acide lévulinique ou un mélange de ceux-ci, et
le sucre étant le facteur limitant de la fermentation dans le milieu de sélection toxique et dans le milieu de sélection non toxique,
où la concentration de l'acide organique non ionisé dans le milieu de sélection toxique est une concentration qui entraîne une diminution de la quantité d'alcool produit à l'instant t_{max ref} par la souche de référence numéro 1-4071 de 25% à 75%, de préférence une diminution de 40% à 75%, par rapport à la quantité maximale d'alcool produite par ladite souche de référence numéro 1-4071 à l'instant t_{max ref} dans le milieu de sélection non toxique, où l'instant t_{max ref} est l'instant où la quantité maximale d'alcool produit par ladite souche de référence numéro 1-4071 est atteinte dans le milieu de sélection non toxique,
- une étape de sélection d'au moins une souche de levure dont la quantité d'alcool produit à l'instant tₘₐₓ dans le milieu de sélection toxique est supérieure ou égale à 65% de la quantité d'alcool produit à l'instant tₘₐₓ par ladite souche dans le milieu de sélection non toxique, où l'instant tₘₐₓ est l'instant où la quantité maximale d'alcool produit par ladite souche de levure est atteinte dans le milieu de sélection non-toxique, et
- une étape supplémentaire de sélection d'au moins une souche de levure dont la quantité d'alcool produit à l'instant tₘₐₓ dans ledit milieu de sélection toxique est supérieure à la quantité d'alcool produit à l'instant tₘₐₓ par la souche de levure de référence numéro 1-4071 dans ledit même milieu de sélection toxique.

2. Procédé selon la revendication 1, **caractérisé en ce que** le milieu de sélection toxique et le milieu de sélection non toxique sont des milieux synthétiques.

3. Souche de levure obtenue par le procédé selon la revendication 1 ou la revendication 2 et choisie parmi la souche de *Saccharomyces cerevisiae* déposée auprès de la CNCM sous le numéro 1-4264, la souche de *Saccharomyces cerevisiae* déposée auprès de la CNCM sous le numéro 1-4265, la souche de *Saccharomyces cerevisiae* déposée auprès de la CNCM sous le numéro 1-4411 et la souche de *Saccharomyces cerevisiae* déposée auprès de la CNCM sous le numéro 1-4412.

4. Levure obtenue par culture d'une souche de levure selon la revendication 3.

5. Utilisation d'une levure selon la revendication 4, pour la production d'alcool.

6. Procédé de production d'alcool comprenant une étape de fermentation d'au moins une levure selon la revendication 4 dans un milieu de fermentation.

7. Procédé selon la revendication 6, **caractérisé en ce que** ledit milieu de fermentation est un milieu de fermentation toxique.

8. Procédé selon la revendication 7, **caractérisé en ce que** ledit milieu de fermentation toxique comprend une eau recyclée et/ou de la mélasse.

9. Procédé selon la revendication 6 ou la revendication 7, comprenant une étape de recyclage partiel ou total du milieu de fermentation.

## Patentansprüche

1. Verfahren zur Selektion eines gegen ein toxisches Medium resistenten Hefestamms, umfassend:
- einen Schritt der Vorauswahl wenigstens eines Hefestamms, für den die maximal hergestellte Alkoholmenge in einem nicht toxischen Selektionsmedium unter nicht begrenzenden Zuckerbedingungen größer als oder gleich 92% der maximal hergestellten Alkoholmenge in dem nicht toxischen Selektionsmedium ist, durch den bei der CNCM am 4. September 2008 unter der Nummer I-4071 hinterlegten Referenzstamm,
- einen Fermentationsschritt des wenigstens einen vorgewählten Hefestamms in einem toxischen Selektionsmedium und in einem nicht-toxischen Selektionsmedium,
wobei die anfängliche Zusammensetzung des toxischen Selektionsmediums die anfängliche Zusammensetzung des nicht toxischen Selektionsmediums ist, plus wenigstens eine nicht ionisierte organische Säure, gewählt aus Essigsäure, Milchsäure, Ameisensäure, Levulinsäure oder deren Mischung, und
wobei der Zucker der begrenzende Faktor der Fermentation in dem toxischen Selektionsmedium und in dem nicht toxischen Selektionsmedium ist,
wobei die Konzentration der nicht ionisierten organischen Säure in dem toxischen Selektionsmedium eine Konzentration ist, die zu einer Verringerung der hergestellten Alkoholmenge zum Zeitpunkt t_{max ref} durch den Referenzstamm Nummer I-4071 von 25% bis 75% führt, bevorzugt zu einer Verringerung von 40% bis 75%, bezüglich der maximal zum Zeitpunkt t_{max ref} durch den Referenzstamm Nr. I-4071 in dem nicht-toxischen Selektionsmedium hergestellten Alkoholmenge, wobei der Zeitpunkt t_{max ref} der Zeitpunkt ist, zu dem die durch den Referenzstamm Nummer I-4071 maximal hergestellte Alkoholmenge in dem nicht-toxischen Selektionsmedium erreicht ist,
- einen Selektionsschritt wenigstens eines Hefestamms, für den die zum Zeitpunkt t_{max ref} in dem toxischen Selektionsmedium hergestellte Alkoholmenge größer als oder gleich 65% der maximal zum Zeitpunkt t_{max ref} durch den Stamm in dem nicht-toxischen Selektionsmedium hergestellten Alkoholmenge ist, wobei der Zeitpunkt t_{max ref} derjenige Zeitpunkt ist, an dem die durch den Hefestamm maximal hergestellte Alkoholmenge in dem nicht-toxischen Selektionsmedium erreicht ist, und
- einen zusätzlichen Selektionsschritt wenigstens eines Hefestamms, für den die zum Zeitpunkt t_{max ref} in dem toxischen Selektionsmedium hergestellte Alkoholmenge größer ist als die zum Zeitpunkt t_{max ref} durch den Hefereferenzstamm Nummer I-4071 in dem gleichen toxischen Selektionsmedium hergestellte Alkoholmenge.

2. Verfahren nach Anspruch 1, **dadurch gekennzeichnet, dass** das toxische Selektionsmedium und das nicht-toxische Selektionsmedium synthetische Medien sind.

3. Durch das Verfahren nach Anspruch 1 oder Anspruch 2 gewonnener Hefestamm, welcher gewählt ist aus dem Stamm *Saccharomyces cerevisiae* mit der CNCM-Hinterlegungsnummer I-4264, dem Stamm *Saccharomyces cerevisiae* mit der CNCM- Hinterlegungsnummer I-4265, dem Stamm *Saccharomyces cerevisiae* mit der CNCM- Hinterlegungsnummer I-4411 und dem Stamm *Saccharomyces cerevisiae* mit der CNCM- Hinterlegungsnummer I-4412.

4. Hefe, welche durch die Kultur eines Hefestamms nach Anspruch 3 erhalten wird.

5. Verwendung einer Hefe nach Anspruch 4 für die Herstellung von Alkohol.

6. Verfahren der Alkoholherstellung, umfassend einen Schritt der Fermentation wenigstens einer Hefe nach Anspruch 4 in einem Fermentationsmedium.

7. Verfahren nach Anspruch 6, **dadurch gekennzeichnet, dass** das Fermentationsmedium ein toxisches Fermentationsmedium ist.

8. Verfahren nach Anspruch 7, **dadurch gekennzeichnet, dass** das toxische Fermentationsmedium wiederaufbereitetes Wasser und/oder Melasse umfasst.

9. Verfahren nach Anspruch 6 oder Anspruch 7, welches einen Schritt der teilweisen oder vollständigen Wiederaufbereitung des Fermentationsmediums umfasst.

## Claims

1. Method of selecting a yeast strain that is resistant to a toxic medium, comprising:
- a step of pre-selection of at least one yeast strain for which the maximum amount of alcohol produced, in a non-toxic selection medium under non-limiting sugar conditions, is greater than or equal to 92% of the maximum amount of alcohol produced, in the non-toxic selection medium, by the reference strain filed on 4 September 2008 at the CNCM [French National Collection of Microorganism Cultures] under number I-4071,
- a step of fermentation, in a toxic selection medium and in a non-toxic selection medium, of the at least one pre-selected yeast strain,
the initial composition of the toxic selection medium being the initial composition of the non-toxic selection medium to which has been added at least one non-ionized organic acid chosen from acetic acid, lactic acid, formic acid, levulinic acid or a mixture thereof, and
the sugar being the limiting factor of the fermentation in the toxic selection medium and in the non-toxic selection medium,
wherein the concentration of the non-ionized organic acid in the toxic selection medium is a concentration which leads to a decrease in the amount of alcohol produced at time t_{max ref} by the reference strain number 1-4071 of 25% to 75%, preferably a decrease of 40% to 75%,
relative to the maximum amount of alcohol produced by said reference strain of number 1-4071 at time t_{max ref} in the non-toxic selection medium, wherein the time t_{max ref} is the time at which the maximum amount of alcohol produced by said reference strain number 1-4071 is reached in the non-toxic selection medium,
- a step of selection of at least one yeast strain for which the amount of alcohol produced at time tₘₐₓ in the toxic selection medium is greater than or equal to 65% of the amount of alcohol produced at time tₘₐₓ by said strain in the non-toxic selection medium, wherein the time tₘₐₓ is the time at which the maximum amount of alcohol produced by said yeast strain is reached in the non-toxic selection medium, and
- an additional step of selection of at least one yeast strain for which the amount of alcohol produced at time tₘₐₓ in said toxic selection medium is greater than the amount of alcohol produced at time tₘₐₓ by the reference yeast strain number 1-4071 in said same toxic selection medium.

2. Method according to Claim 1, **characterized in that** the toxic selection medium and the non-toxic selection medium are synthetic media.

3. Yeast strain obtained by the method according to Claim 1 or Claim 2 and chosen from the *Saccharomyces cerevisiae* strain deposited with the CNCM under number 1-4264, the *Saccharomyces cerevisiae* strain deposited with the CNCM under number 1-4265, the *Saccharomyces cerevisiae* strain deposited with the CNCM under number 1-4411 and the *Saccharomyces cerevisiae* strain deposited with the CNCM under number 1-4412.

4. Yeast obtained by culture of a yeast strain according to Claim 3.

5. Use of a yeast according to Claim 4, for producing alcohol.

6. Method of producing alcohol, comprising a step of fermentation of at least one yeast according to Claim 4 in a fermentation medium.

7. Method according to Claim 6, **characterized in that** said fermentation medium is a toxic fermentation medium.

8. Method according to Claim 7, **characterized in that** said toxic fermentation medium comprises recycled water and/or molasses.

9. Method according to Claim 6 or Claim 7, comprising a step of partial or total recycling of the fermentation medium.
